# EUROPEAN PATENT SPECIFICATION

(11) **EP 3 268 138 B1**
(45) Date of publication and mention of the grant of the patent: **24.04.2019**
(21) Application number: 16709756.7
(22) Date of filing: 10.03.2016
(51) Int. Cl.: B08B 9/027, B08B 9/032, A61L 2/07, A61L 2/18, B67C 3/00

(54) **METHOD FOR REDUCING WATER CONSUMPTION OF A SYSTEM FOR PROCESSING A LIQUID OR A SEMI-LIQUID FOOD PRODUCT**
VERFAHREN ZUR VERRINGERUNG DES WASSERVERBRAUCHS EINES SYSTEMS ZUR VERARBEITUNG EINES FLÜSSIGEN ODER HALBFLÜSSIGEN LEBENSMITTELPRODUKTS
PROCÉDÉ DE RÉDUCTION DE LA CONSOMMATION D'EAU D'UN SYSTÈME POUR LE TRAITEMENT D'UN PRODUIT ALIMENTAIRE LIQUIDE OU SEMI-LIQUIDE

(30) Priority: 13.03.2015 SE 1550306
(43) Date of publication of application: 17.01.2018
(73) Proprietor: Tetra Laval Holdings & Finance S.A., 1009 Pully (CH)
(72) Inventor: TOFT, Pernilla, S-244 39 Kävlinge (SE); STJERNBERG, Göran, S-224 75 Lund (SE); JENSINGER, Peter, S-244 66 Furulund (SE)
(74) Representative: Tetra Pak - Patent Attorneys SE
(86) International application number: PCT/EP2016/055119
(87) International publication number: WO 2016/146471

(56) References cited:
- WO-A1-2005/102546
- WO-A1-2014/044600
- US-A1- 2004 118 291

## Description

### Technical field

The present invention generally relates to a method for reducing water consumption of a system for processing a liquid or semi-liquid food product, after an interruption of such a processing.

### Background art

In order to meet increasing demands for reducing water consumption and energy efficiency in food processing, food processing companies are always looking into new ways of reducing water consumption and energy needed for the processing of food.

Food processing systems naturally need to be clean and free from unwanted bacteria to meet food safety regulations. It is therefore required to regularly stop the food processing system for cleaning and sterilizing. This is normally done by emptying the system, flush it with water and circulate with water mixed with cleaning chemicals. The cleaning water, which is often very aggressive, is then flushed out of the system by clean water. The clean water is then heated to sterilize the system. The sterilizing water is then emptied of the system before the system again is taken into service for continued food processing.

Also when the food processing machine is stopped to change the production to a different product, the system has to be flushed with water to avoid mixing of the products. Additional prior art is reflected by patent document WO2005/102546 A1.

In these processes, the food processing system is flushed at least twice with water, and since the food processing system is rather large in volume, each flushing may use several cubic meters of fresh water. In many parts of the world, fresh water is a precious natural resource that is in shortage. The used water is also drained to the sewage system creating an increased volume of sewage water that someone has to deal with.

There is thus a need for improving the state of the art and provide methods of reducing the water consumption in food processing systems.

### Summary of the invention

It is an object of the present invention to improve the current state of the art, to solve the above problems, and to provide an improved method for reducing the water consumption in food processing systems. These and other objects are achieved by a method for reducing water consumption of a system for processing a liquid or semi-liquid food product, after an interruption of said processing. Such an interruption may for example consist of maintenance of the system or replacement of the second sub-concentration when a new food product is to be processed. The food product is a composition of a first sub-composition having a low concentration and a second sub-composition having a high concentration, wherein said high concentration is greater than said low concentration. The method comprises the steps of pushing said first sub-composition, said second sub-composition and said food product out of the system using water until the system substantially contains only water, sterilizing the system by heating the water present in the system, and forming a new food product by adding a new second sub-composition to the sterile water, thereby using said sterile water as said first sub-composition. The advantages of such a method are apparent for a person skilled in the art. It is not only an easy and time efficient way to handle the problem of cleaning the system during maintenance or change of sub-composition, it is also a brilliant way to save enormous amounts of water during the processing of the food product. In conventional processing methods, the water that is used to flush and clean the system is always discarded or drained after the cleaning process while the present method will ensure that all of that water that is clean is reused for the next food product to be processed.

The step of sterilizing the system may comprise heating the water present in the system to above 100 °C and circulating the water in the system. This is a time and cost effective way of sterilizing the system.

Before the step of sterilizing the system, the method may further comprise the step of cleaning the system by adding chemicals to the water, and by pushing the water containing cleaning chemicals out of the system using clean water until the system substantially contains only water. Thus, the cleaning process of the system is further enhanced. When system once again is filled with clean water only, the processing of the food product may be resumed, or the processing of a new food product may be started. The cleaning chemicals may typically consist of acids and/or lye. In order to even further increase the efficiency of the cleaning process, the step of cleaning the system may further comprise circulating the cleaning chemicals in the system.

Basically any food product may be processed in this type of system using the present method. However, the second sub-composition is typically chosen from the group consisting of: juice concentrate, milk concentrate or other premixes comprising for instance aromas, concentrates, soy milk, rice based milk, grain based milk and nut based milk.

The method may further comprise the step of adding a new first sub-composition when said sterile water is consumed. That is to say, when the sterile water that is consumed, fresh water will be pumped into the system and used as the first sub-composition. Should, however, the processing of the food product be stopped or interrupted before all of the sterile water has been used up, the method may further comprise the step of buffering any excessive sterile water. Naturally, this step is used in order to avoid disposal or drainage of the sterile water.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

### Brief description of the drawings

The above objects, as well as additional objects, features and advantages of the present invention, will be more fully appreciated by reference to the following illustrative and non-limiting detailed description of preferred embodiments of the present invention, when taken in conjunction with the accompanying drawings, wherein:
Fig. 1 is a block scheme over a method for reducing water consumption of a system for processing a liquid or semi-liquid food product.
Fig. 2 is a schematic drawing of a system for processing a liquid or semi-liquid food product according to the invention connected to an aseptic drink system.

### Detailed description of preferred embodiments of the invention

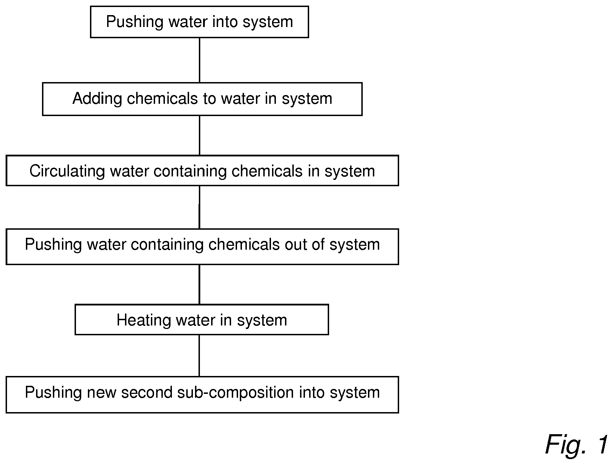

In Fig. 1, a method for reducing water consumption of a system for processing a liquid or semi-liquid food product according to one exemplary embodiment of the invention is illustrated.

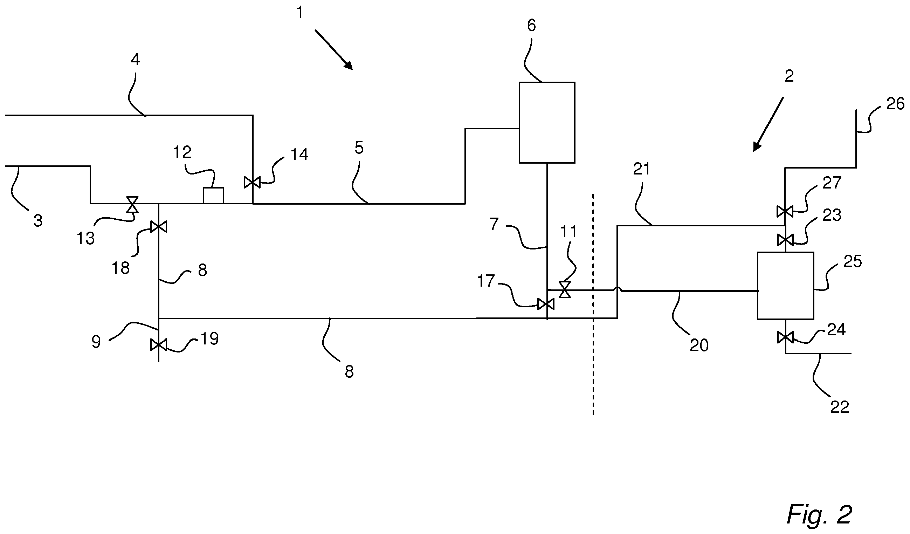

Fig. 2 is a schematic drawing of a system 1 for processing a liquid or semi-liquid food product. The food product is mixed by a first sub-composition having a low concentration and a second sub-composition having a high concentration. The first sub-composition is in the simplest form pre-treated water arriving from a water supply (not shown) through the conduit 3. The second sub-composition, which is normally a concentrate of the food product, e.g. concentrated juice, is supplied from a food concentrate supply (not shown) via the conduit 4. Both the water supply and the food concentrate supply may be turned off by shutting the valves 13 and 14, respectively. The food concentrate and water are mixed in the conduit 5, leading to a buffer tank 6 in which the mix is stirred to a homogenous mix of the final food product. A so-called Brix (concentration) meter 12, or any other device achieving the same purpose, for instance a density meter or an IR (infra red) spectrometer, may be used for making sure that the correct amounts of water from the conduit 3 and food product concentrate from the conduit 4 are mixed. The food product is then lead out from the system 1 via the conduit 7. A feed-back loop 8 is also present that may, if the valve 17 and 18 are opened, connect the conduit 7 to the water supply conduit 3. The content of the feed-back loop may also be drained via the conduit 9 if the valve 19 is opened.

In order to provide for that the water from the water supply does not affect the properties of the food product the water may be pre-treated in different ways. For instance, in order to remove insoluble solids sand filtration may be used, in order to remove color active carbone filtration may be used and in order to reduce dissolved salts membrane filtration may be used.

The concentrate of food product is often delivered to the conduit 4 in tanks. In order to prevent micro bacterial growth the tanks may be cooled, for instance by being equipped with cooling jackets or in cool storage rooms.

In Fig. 2 the outlet conduit 7 of the system 1 and the feed-back loop 8 are connected to an aseptic heat treatment system with an automated control of processing parameters to safeguard production under aseptic conditions. The aseptic heat treatment system contains a further buffer tank 25. The conduit 21 is connected from the further buffer tank 25 to the feed-back loop 8 of the system 1 and the conduit 20 is connected from the outlet conduit 7 of the system 1 to the further buffer tank 25 of the aseptic heat treatment system. A valve 23 controls may open and close the connection from the further buffer tank 25 to the feed-back loop 8. A conduit 22 serves as an outlet from the further buffer tank 25. The outlet 22 may be closed by closing the valve 24. The aseptic heat treatment system 2 further comprises a fresh water inlet conduit 26 for pre-treated water having a valve 27 for opening and closing the conduit 26.

When the system is emptied for cleaning, the valve 14 is closed so that the concentrate is shut off. And the water supply is shut off with the valve 13. The buffer tank 6 is then allowed to be emptied before the system is flushed with water by opening the valve 13 again. If only the system 1 is intended to be cleaned the valve 17 is opened and valve 11 is closed to disconnect the aseptic drink system and to connect the outlet 7 from the buffer tank 6 to the feed-back loop 8 so that water may be circulated in the system 1. When the system contains only water, cleaning chemicals are introduced in the system via the fresh water intake. The water containing cleaning chemicals are circulated in the system during a pre-determined time period to clean all parts exposed to the food product. When the cleaning cycle is finished, the system is flushed with fresh water by pushing all of the water containing chemicals out from the system 1. When only fresh water is present in the system, the water is again circulated and heated to about 95-140°C to sterilize the system 1 for a second pre-determined time period. When the system is sterile, it may again be used for food production. The sterile water standing in the system is however not drained, but instead used as the first sub-composition having a low concentration so as to re-use the clean and sterile water that was used for sterilizing the system 1. If the amount of sterile water is too large, the water may be collected from the valve 19 for temporary storage. When the sterile water is all used as the first sub-composition of the food product, the intake valve 13 for the pre-treated water is again opened and valve 18 of the feed-back loop 8 is closed.

In a further aspect of the invention the feed-back circulation of the system is achieved by looping also the aseptic heat treatment system 2. The valve 23 is opened to allow the conduit 21 to feed the feed-back loop conduit 8. Instead of using the water from the conduit 3 for pushing out food product or cleaning water from the system, a fresh water intake via conduit 26 of the aseptic heat treatment system 2 may be used by opening the valve 27.

When the food production is resumed after an interruption, new food product that is produced will push water in front of it. The first part of the produced food product will then mix with the water in front of it so that the first part of the produced food product will be a diluted product. Until the produced food product reaches the valve 17 or the valve 23 if a aseptic drink system is attached, the valve 17 or 23, respectively, will be held open (while keeping valve 11 or 24, respectively, closed) so that the water that is pushed in front of the food is reused as the first sub-composition in accordance with the present invention. Also the diluted product is rejected into the feed-back loop 8 since it is diluted and not useful as end food product. When all of the diluted product has passed the valve 17 or 23, respectively, the valve 17 or 23 is closed and the valve 11 or 24, respectively, is opened to feed the un-diluted food product to an outlet, e.g. the conduit 22. Since the Brix meter 12 will sense any concentration of food product present in the water that is fed back via the feed-back loop 8 as the first sub-composition, the system will be able to handle that some of the first sub-composition already contains a low concentration of the food product by simply reducing the amount of added concentrate accordingly.

It is understood that other variations in the present invention are contemplated. Accordingly, it is appropriate that the scope of the invention be construed broadly in a manner consistent with the appendend claims.

## Claims

1. Method for reducing water consumption of a system (1) for processing a liquid or semi-liquid food product, after an interruption of said processing,
said food product being a composition of a first sub-composition having a low concentration and a second sub-composition having a high concentration, wherein said high concentration is greater than said low concentration, said method comprising the steps of:
pushing said first sub-composition, said second sub-composition and said food product out of the system (1) using water until the system (1) contains only water,
sterilizing the system (1) by heating the water present in the system (1), **characterized in that** the sterile water standing in the system is not drained and by
forming a new food product by adding a new second sub-composition to the sterile water, thereby using said sterile water as said first sub-composition having a low concentration.

2. Method according to claim 1, wherein the step of sterilizing the system (1) comprises heating the water present in the system (1) to 95-140 °C and circulating the water in the system (1).

3. Method according to any one of the preceding claims, further comprising the step of:
before the step of sterilizing the system (1), cleaning the system (1) by adding chemicals to the water, and pushing the water containing cleaning chemicals out of the system (1) using clean water until the system (1) contains only water.

4. Method according to claim 3, wherein said cleaning chemicals are acids and lye.

5. Method according to claim 3, wherein said step of cleaning the system (1) further comprises circulating said cleaning chemicals in the system (1).

6. Method according to any one of the preceding claims, wherein said interruption occurs due to maintenance of the system (1) or due to replacement of said second sub-composition.

7. Method according to any one of the preceding claims, wherein said second sub-composition is chosen from the group consisting of: juice concentrate, milk concentrate.

8. Method according to any one of the preceding claims, further comprising the step of adding a new first sub-composition when said sterile water is consumed.

9. Method according to any one of the preceding claims, further comprising the step of buffering any excessive sterile water.

## Patentansprüche

1. Verfahren zum Verringern eines Wasserverbrauchs eines Systems (1) zur Verarbeitung eines flüssigen oder halbflüssigen Lebensmittelprodukts nach einer Unterbrechung der Verarbeitung,
wobei das Lebensmittelprodukt eine Zusammensetzung aus einer ersten Teilzusammensetzung, die eine niedrige Konzentration aufweist, und einer zweiten Teilzusammensetzung ist, die eine hohe Konzentration aufweist, wobei die hohe Konzentration größer als die niedrige Konzentration ist, wobei das Verfahren die folgenden Schritte umfasst:
Drücken der ersten Teilzusammensetzung, der zweiten Teilzusammensetzung und des Lebensmittelprodukts mithilfe von Wasser aus dem System (1), bis das System (1) nur noch Wasser enthält,
Sterilisieren des Systems (1), indem das in dem System (1) vorhandene Wasser erwärmt wird, **dadurch gekennzeichnet, dass** das sterile Wasser, das in dem System steht, nicht abgelassen wird, und **gekennzeichnet durch** ein Bilden eines neuen Lebensmittelprodukts, indem eine neue zweite Teilzusammensetzung zu dem sterilen Wasser hinzugefügt wird, wodurch das sterile Wasser als die erste Teilzusammensetzung verwendet wird, die eine niedrige Konzentration aufweist.

2. Verfahren nach Anspruch 1,
wobei der Schritt des Sterilisierens des Systems (1) ein Erwärmen, des in dem System (1) vorhandenen Wassers auf 95 bis 140 °C und ein Zirkulieren des Wassers in dem System (1) umfasst.

3. Verfahren nach einem der vorhergehenden Ansprüche, das außerdem den folgenden Schritt umfasst:
vor dem Schritt des Sterilisierens des Systems (1), Reinigen des Systems (1), indem Chemikalien zu dem Wasser hinzugefügt werden, und Drücken des Wassers, das die Reinigungschemikalien enthält, mithilfe von sauberem Wasser aus dem System (1), bis das System (1) nur noch Wasser enthält.

4. Verfahren nach Anspruch 3, wobei die Reinigungschemikalien Säuren und Laugen sind.

5. Verfahren nach Anspruch 3, wobei der Schritt des Reinigens des Systems (1) außerdem ein Zirkulieren der Reinigungschemikalien in dem System (1) umfasst.

6. Verfahren nach einem der vorhergehenden Ansprüche, wobei die Unterbrechung aufgrund der Wartung des Systems (1) oder aufgrund des Austauschs der zweiten Teilzusammensetzung auftritt.

7. Verfahren nach einem der vorhergehenden Ansprüche, wobei die zweite Teilzusammensetzung aus der Gruppe ausgewählt wird, die besteht aus: Saftkonzentrat und Milchkonzentrat.

8. Verfahren nach einem der vorhergehenden Ansprüche, das außerdem den Schritt eines Hinzufügens einer neuen ersten Teilzusammensetzung umfasst, wenn das sterile Wasser aufgebraucht ist.

9. Verfahren nach einem der vorhergehenden Ansprüche, das außerdem den Schritt eines Zwischenspeicherns von überschüssigem sterilem Wasser umfasst.

## Revendications

1. Procédé pour réduire la consommation d'eau d'un système (1) afin de traiter un produit alimentaire liquide ou semi-liquide, après une interruption dudit traitement,
ledit produit alimentaire étant une composition d'une première sous-composition ayant une faible concentration et d'une deuxième sous-composition ayant une forte concentration, ladite forte concentration étant plus grande que ladite faible concentration, ledit procédé comprenant les étapes consistant à :
pousser ladite première sous-composition, ladite deuxième sous-composition et ledit produit alimentaire hors du système (1) en utilisant de l'eau jusqu'à ce que le système (1) ne contienne que de l'eau,
stériliser le système (1) en chauffant l'eau présente dans le système (1), **caractérisé en ce que** l'eau stérile se trouvant dans le système n'est pas évacuée, et en formant un nouveau produit alimentaire en ajoutant une nouvelle deuxième sous-composition à l'eau stérile, en utilisant ainsi ladite eau stérile comme ladite première sous-composition ayant une faible concentration.

2. Procédé selon la revendication 1, dans lequel l'étape de stérilisation du système (1) comprend le chauffage de l'eau présente dans le système (1) jusqu'à 95-140 °C et la circulation de cette eau dans le système (1).

3. Procédé selon l'une quelconque des revendications précédentes, comprenant en outre l'étape consistant à :
nettoyer le système (1), avant l'étape de stérilisation du système (1), en ajoutant des produits chimiques à l'eau, et en poussant l'eau contenant des produits chimiques de nettoyage hors du système (1) en utilisant de l'eau propre jusqu'à ce que le système (1) ne contienne que de l'eau.

4. Procédé selon la revendication 3, dans lequel lesdits produits chimiques de nettoyage sont des acides et de la lessive de soude.

5. Procédé selon la revendication 3, dans lequel ladite étape de nettoyage du système (1) comprend en outre la circulation desdits produits chimiques de nettoyage dans le système (1).

6. Procédé selon l'une quelconque des revendications précédentes, dans lequel ladite interruption se produit à cause de la maintenance du système (1) ou à cause du remplacement de ladite deuxième sous-composition.

7. Procédé selon l'une quelconque des revendications précédentes, dans lequel ladite deuxième sous-composition est choisie parmi le groupe comprenant : du jus concentré, du lait concentré.

8. Procédé selon l'une quelconque des revendications précédentes, comprenant en outre l'étape consistant à ajouter la nouvelle première sous-composition lorsque ladite eau stérile est consommée.

9. Procédé selon l'une quelconque des revendications précédentes, comprenant en outre l'étape consistant à stocker l'eau stérile excessive dans un réservoir tampon.
